# EUROPEAN PATENT APPLICATION

(11) **EP 4 006 144 A1**
(43) Date of publication of application: **01.06.2022**
(21) Application number: 19940120.9
(22) Date of filing: 31.07.2019
(51) Int. Cl.: C12N 5/077, C12N 5/0775, A61K 35/28

(54) **THERAPEUTIC MEDICINE FOR FIBROSIS, INFLAMMATION, AND/OR AGE-RELATED DISEASES**

(71) Applicant: Shanghai Santeja H & M Tech. Development Inc, Shanghai 201203 (CN); Exrhera Limited, Hong Kong 999077 (HK); Takahiro, Ochiya, Tokyo 104-0053 (JP)
(72) Inventor: TAKAHIRO, Ochiya, Tokyo 104-0053 (JP)
(74) Representative: Greiner, Elisabeth
(86) International application number: PCT/CN2019/000152
(87) International publication number: WO 2021/016727

(57) **Abstract**

An object of the present invention is to provide a therapeutic agent and a preventive agent which exert a therapeutic effect on fibrotic diseases such as pulmonary fibrosis, inflammatory diseases and / or aging-associated diseases. Focusing on exosomes, the present inventors have repeatedly studied on the pathology and treatment methods of fibrotic diseases such as pulmonary fibrosis, inflammatory diseases, and aging-associated diseases, and as a result thereof, it was found that the exosomes derived from mesenchymal stem cells (MSC) have excellent therapeutic effects and/or preventive effects on the above diseases. Accordingly, the present invention relates to a therapeutic or prophylactic agent and a pharmaceutical composition comprising the therapeutic and/or prophylactic agent, which is a therapeutic or prophylactic agent for at least one of a fibrotic disease, an inflammatory disease, and an aging-associated disease, and comprises exosomes derived from mesenchymal stem cells.

## Description

### Technical field

The present disclosure relates to a therapeutic agent and a prophylactic agent for at least one disease of fibrotic diseases, inflammatory diseases, and aging diseases, and a treatment method or a preventive method using the therapeutic or prophylactic agent.

### Background

Tissue fibrosis refers to the excess formation of fibrous connective tissue produced during the repair or healing of organs or tissues. Such tissue fibrosis may occur in various organs as fibrotic diseases, for example, known pulmonary fibrosis and liver cirrhosis. Although the number of patients with fibrotic diseases has risen to hundreds of thousands in Japan, there is still no effective treatment.

Pulmonary fibrosis is a representative fibrotic disease, and also known as an inflammatory disease or an aging disease. Idiopathic pulmonary fibrosis (IPF), of which the cause cannot be identified, has a life expectancy of 3 to 5 years after diagnosis. The prognosis is extremely poor. The incidence of IPF in Japan is about 20 per 100,000 individuals. Antifibrotic drugs such as pirfenidone and nintedanib, steroids, immunosuppressants, *etc.* can be used in treatment for pulmonary fibrosis (Non-Patent References 1 to 2), but resulting in limited effect.

Chronic obstructive pulmonary disease (COPD) refers to the general term for diseases that used to be called chronic bronchitis and emphysema. It is an inflammatory disease in the lungs caused by long-term inhalation/exposure to harmful substances mainly including tobacco smoke. It is a lifestyle disease that occurs in middle-aged and elderly people on the background of smoking habits. COPD is estimated to exist in 8.6% of the population over 40 years of age, approximately 5.3 million people (NICE study). Most patients are considered to be in an undiagnosed and untreated state. It is the 9^{th} cause of death among all diseases and for men the 7^{th}. According to WHO, it is considered to be an important disease that will rise to the 3^{rd} cause of death in the world by 2020.

Smoking is thought to be the biggest cause of COPD. 15 to 20% of smokers develop COPD. Inhalation of tobacco smoke causes inflammation of the bronchial tubes in the lungs, causing coughing, excessive sputum, or narrowing of the bronchial tubes, resulting in reduced air flow. In addition, the function of taking in oxygen and expelling carbon dioxide will be reduced, if the grape cluster-shaped pouches (*i.e.* alveolus) located deep in the branches of the bronchi are destroyed and become emphysema. These changes are believed to coexist with COPD and are not reversible even after treatment.

IPF (idiopathic pulmonary fibrosis) is also known as an unexplained interstitial pneumonia (idiopathic interstitial pneumonia), and is the disease with the highest incidence among idiopathic interstitial pneumonia. The incidence of IPF in men is higher than that in women, and the incidence of IPF increases with age. IPF may also become a cause of lung cancer due to progressive deterioration of the interstitial fibrosis.

For these respiratory organs, the main symptoms are dyspnea during exercise that feels breathless when walking or moving up and down stairs, chronic cough, and abundant expectoration. Some patients also exhibit symptoms including wheezing and paroxysmal dyspnea such as asthma.

The goals of COPD management are: (1) improvement of symptoms and quality of life; (2) improvement and maintenance of exercise capacity and physical activity; (3) prevention of disease deterioration; (4) inhibition of disease progression; (5) prevention and treatment of systemic and pulmonary complications; (6) improvement of life prognosis. If the patient continues to smoke, the deterioration of respiratory function will be accelerated. Therefore, smoking ban is the basis of treatment. Influenza and pneumococcal vaccines are recommended to avoid deterioration of the disease. The core of drug therapy is bronchodilators (anticholinergic drugs/β2 receptor agonists/theophylline drugs). In terms of treatment effects and side effects, inhaled drugs are recommended, mainly including inhaled β2 receptor agonists and inhaled anticholinergic drugs for long-term bronchial expansion. Inhaled steroids may be used in case of severe airflow obstruction and repeated deteriorations of the condition. It has been reported that a combination of long-acting β2 receptor agonists and inhaled steroids is also useful. The core of non-drug therapy is respiratory rehabilitation (breathing training such as mouth breathing and abdominal breathing/exercise therapy/nutrition therapy, etc.). In a case where hypoxemia progresses, home oxygen therapy can be introduced. Furthermore, in a case where respiratory failure progresses, a ventilation assist therapy is sometimes performed using a small artificial respirator and a face mask to assist breathing. Depending on the case, a surgical operation (lung volume reduction surgery) is also performed to remove the hyperinflated lung.

However, COPD and IPF are extremely difficult to be treated and are considered to be diseases that are difficult to treat (cure) completely so far. Therefore, various treatments aim to suppress the progression of the diseases. Therefore, it is eager to develop a new treatment method for respiratory diseases that will continue to increase in the future.

### Prior art reference

### Non-patent References

Non-Patent Reference 1: Azuma A, et al., Am. J. Respir. Crit. Care Med., 2005, 171, 1040~1047.
Non-Patent Reference 2: Richeldi L et al., N. Engl. J. Med., 2011, 22, 365(12), 1079-87.

### Summary of the invention

### Problems to be solved by the invention

In one embodiment, a problem of the present disclosure is to provide a therapeutic and/or prophylactic agent for fibrotic diseases such as pulmonary fibrosis, inflammatory diseases, and/or aging diseases.

### Means to solve the problems

The inventors of the present disclosure have repeatedly studied the pathology and treatment methods of fibrotic diseases such as pulmonary fibrosis, inflammatory diseases, and aging diseases, and first to show the world: exosomes derived from airway epithelial cells stimulated by smoking can be a cause of the pathology of respiratory diseases comprising COPD and IPF. Subsequently, further experiments focusing on exosomes were carried out, and exosomes derived from mesenchymal stem cells (hereinafter referred to as "MSCs") have been found to have superior therapeutic and/or preventive effects against the above-mentioned diseases. In particular, the inventors completed the present disclosure by discovering the fact that inhalation of exosomes of human MSCs through the airway can treat these respiratory diseases and even treat asthma.

Therefore, the present disclosure relates to the following inventions.
(1) A therapeutic and/or prophylactic agent for at least one disease of fibrotic diseases, inflammatory diseases, and aging diseases, comprising exosomes derived from MSCs.
(2) The therapeutic or prophylactic agent according to (1), wherein
   the MSCs are cells isolated from bone marrow, adipose tissue, umbilical cord or peripheral blood.
(3) The therapeutic or prophylactic agent according to (1), wherein
   the MSCs are cells isolated from pluripotent stem cells (including iPS cells and ES cells).
(4) The therapeutic or prophylactic agent according to any one of (1) to (3), wherein
   the MSCs are human cells.
(5) The therapeutic or prophylactic agent according to any one of (1) to (4), wherein
   the diseased site of the disease is lung.
(6) The therapeutic or prophylactic agent according to (5), wherein
   the disease is selected from the group consisting of pulmonary fibrosis, COPD, bronchial asthma, ARDS (acute respiratory distress syndrome), pulmonary hypertension, radiation pneumonitis, pulmonary sarcoidosis, alveolar hemorrhage accompanied by collagen disease, or interstitial pneumonia.
(7) The therapeutic or prophylactic agent according to any one of (1) to (6), which is an inhalant.
(8) The therapeutic or prophylactic agent according to any one of (1) to (7), which is used for intratracheal administration or transpulmonary administration.

### Effect of the invention

According to the present disclosure, a therapeutic and/or prophylactic agent for fibrotic diseases, inflammatory diseases, and/or aging diseases may be provided.

### Brief description of the drawings

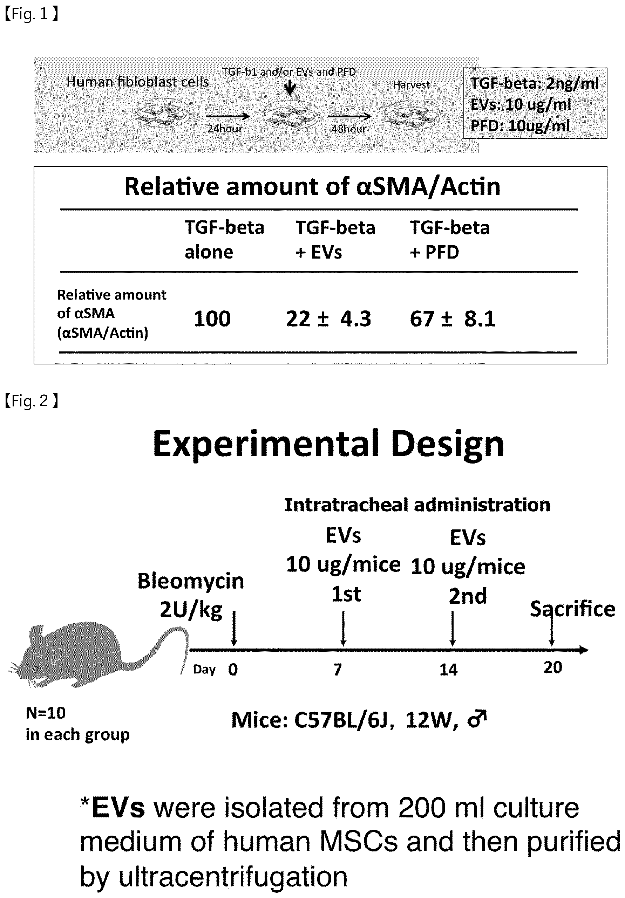

Fig. 1 is a graph showing the experimental method and results of the myofibroblast differentiation inhibition assay performed *in vitro.* In the table, results of quantification of the expression level (mRNA) of αSMA in cases where TGFβ (2 ng/mL), TGFβ (2 ng/mL) + MSC-derived exosomes (10 µg/mL), TGFβ (2 ng/mL) + pirfenidone (PFD) (10 µg/mL) were added are shown in order from the left.
Fig. 2 is a dosing schedule showing the evaluation of the therapeutic effect using the animal model of Example 3.

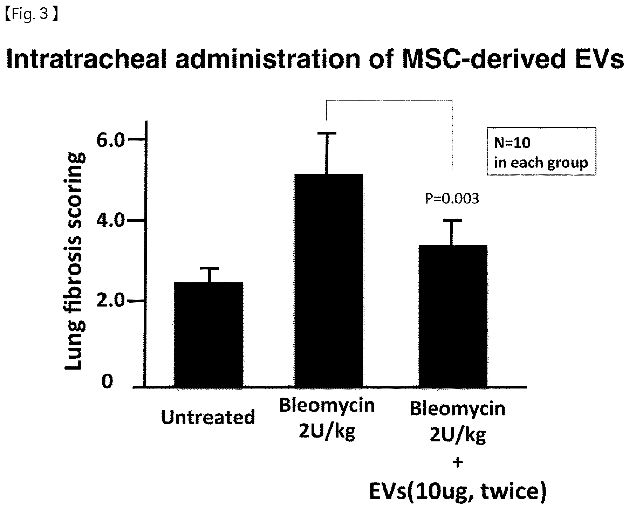

FIG. 3 is a graph showing the results of the animal experiment in Example 3. From left to right: a group not administered with bleomycin and exosomes; a group administered with bleomycin but no exosomes; and a group administered with both bleomycin and exosomes. The vertical axis represents the lung fibrosis score achieved by Masson trichrome staining.

### Detailed description

### 1. Therapeutic and/or prophylactic agent for diseases

In one embodiment, the present disclosure relates to a therapeutic and/or prophylactic agent for at least one disease of fibrotic diseases, inflammatory diseases, and aging diseases, comprising exosomes derived from MSCs.

In this specification, the "fibrotic disease" means a disease accompanied by excessive formation of fibrous connective tissue produced during the repair or healing of organs or tissues. Fibrotic diseases are diseases caused by abnormal deposits of scar tissue, or diseases that are accompanied by these disorders. Fibrotic diseases may develop in a variety of tissues. Examples of fibrotic diseases may include, but are not limited to, pulmonary fibrosis, pulmonary hypertension, liver cirrhosis, cardiomyopathy, ischemic heart disease, valvular disease, endomyocardial fibrosis, myocardial fibrosis, arteriosclerosis, renal fibrosis, nephrosclerosis, glomerulosclerosis, scleroderma, retroperitoneal fibrosis, and uterine fibrosis.

In the present specification, "inflammatory disease" means a disease that presents a persistent or prompt inflammatory state, and the symptoms can be improved by alleviating the inflammation. Examples of inflammatory diseases may include, but are not limited to, COPD (chronic obstructive pulmonary disease), bronchial asthma, ARDS (acute respiratory distress syndrome), radiation pneumonia, pulmonary sarcoidosis, viral hepatitis, autoimmune hepatitis, primary biliary cholangitis, primary sclerosing cholangitis, hepatocellular carcinoma, Crohn's disease, ulcerative colitis, acute pancreatitis, chronic pancreatitis, autoimmune pancreatitis, aortitis, Graves' disease, Hashimoto's disease, diabetes, nephritis, collagen disease (e.g., rheumatoid arthritis, SLE (systemic lupus erythematosus), vasculitis, Sjogren's syndrome, arthritis, nodular disease), dermatomyositis, and psoriasis.

In the present specification, "aging disease" means a disease that is caused by aging and is accompanied by a decrease in the homeostasis maintenance function of a tissue or an individual. The representative manifestation of aging is cell aging. The accumulation of aging cells and various mediators secreted therefrom are related to the initiation of chronic inflammation, and in turn are related to structural changes and dysfunctions such as fibrosis. Examples of aging diseases include, but are not limited to, Alzheimer's disease, dementia, Parkinson's disease, spinocerebellar degeneration, and multiple system atrophy.

It should be noted that the above classifications of diseases are not mutually exclusive, and one disease may belong to multiple classifications. For example, primary sclerosing cholangitis and hepatocellular carcinoma may be an inflammatory disease as well as a fibrotic disease simultaneously.

The therapeutic and/or prophylactic agent of the present disclosure comprises, or consists of, or substantially consists of exosomes derived from MSCs. Herein, "derived from mesenchymal stem cells (MSC)" means that exosomes are obtained from MSCs.

In this specification, "exosomes" refer to extracellular vesicles having a diameter of about 20 to 200 nm that are released from various cells. Exosomes are known to have various functions mainly including intercellular communication, antigen presentation, and delivery of proteins and nucleic acids such as mRNAs and miRNAs.

The preparation of exosomes may be carried out using any known method. For example, exosomes may be recovered from the supernatant of a medium by culturing MSCs in the medium. The culture conditions (temperature, duration, *etc.*) may be appropriately selected. For example, the culture temperature may be set to about 20 °C to about 40 °C, about 30 °C to about 40 °C, about 35 °C to about 39 °C, and about 36 °C to about 38 °C, or about 37 °C. The culture duration may be, for example, 6 hours to 7 days, 12 hours to 4 days, 1 day to 3 days, or about 2 days. The culture may be carried out in the presence of CO₂, in which case the CO₂ concentration may be about 2% to about 10%, about 4% to about 6%, or about 5%. In addition, the medium used for culturing may be selected according to the type of cells used. Examples of useful medium include commercially available medium (for example, BEGM, DMEM, MEM, BME, RPMI 1640, Advanced RPMI 1640, F-10, F-12, DMEM-F12, α-MEM, IMDM, MacCOy's 5A medium, mTeSR1 medium or their mixture) or prepared medium. Various additives (for example, serum or serum substitutes, non-essential amino acids, antibiotics such as penicillin and streptomycin) may also be added to the medium. The medium is preferably free of exosomes derived from other components such as serum.

Recovery of exosomes from culture medium may be carried out using any well-known method. Examples include: ultracentrifugation (for example, Thery C., Curr. Protoc. Cell Biol. (2006) Chapter 3: Unit 3.22.), polymer precipitation, immunoprecipitation, FACS, ultrafiltration, gelfiltration, HPLC, and methods involving use of antibodies or lectins to adsorb to a carrier such as beads. In addition, a commercially available kit for exosome isolation may also be used to recover exosomes.

Among the above-mentioned recovery methods, ultracentrifugation is the most commonly used standard method for isolation of exosomes. The centrifugal force of the ultracentrifugation method may be 50000×g or more, 100,000×g or more, or 1,500,000×g or more, and may be 300,000×g or less, 250,000×g or less, or 200,000×g or less. The centrifugation duration may be for example, but not limited to, 30 minutes to 120 minutes, 60 minutes to 90 minutes, or 70 minutes to 80 minutes. In addition, adulterants may also be removed or reduced by filter filtration and/or centrifugal separation at lower centrifugal forces, as needed, prior to centrifugal separation.

Confirmation of the physical properties of recovered exosomes or exosomes may be performed according to well-known methods, for example, visual confirmation by electron microscopy or using NTA (Nano Tracking Analysis) techniques to measure the particle size and number of exosomes. Alternatively, the presence of exosomes may be confirmed by identifying the expression of proteins and/or genes that may be used as markers of the exosomes.

In this specification, MSCs that may be used as a source of exosomes can be obtained, for example, by isolating from bone marrow, adipose tissue, umbilical cord blood, umbilical cord, bone marrow, placenta, or peripheral blood. MSCs have adhesiveness to plastics and are readily to proliferate on petri dishes. Therefore, this property can be used for isolation. For example, after fresh bone marrow is inoculated onto a Petri dish and cultured for several days, MSCs in a form of, for example, fibroblasts begin to appear at the bottom of the Petri dish. After culturing for 5-20 days, MSCs are proliferated in a way that fills the petri dish. CD34(-), CD45(-), CD105(+), and CD29(+) cells can be obtained by FACS or the like from the obtained cell aliquots. Alternatively, cells can be isolated by FACS using more than one MSC-specific markers expressed on mesenchymal cells such as Stro-1, CD271, SSEA-4, CD146, CD49f, CD349SSEA-3, SUSD2, Stro-4, PDGFR-α, Sca-1, and ecto-5'-nucleotidase (CD73).

Alternatively, MSCs may also be obtained through differentiation induction from cells with stronger differentiation ability, such as pluripotent stem cells (including iPS cells and ES cells). For example, it has been reported that MSCs are induced by culturing pluripotent stem cells in the presence of GSK3β inhibitors, TGFβ inhibitors, and insulin (Fukuta M et al. PLoS ONE 9(12): e112291.). Alternatively, MSCs may also be obtained as a commercially available product (Takara Bio Inc. Japan; COSMO BIO CO., Ltd., Japan, *etc.*)*.*

In this specification, the cells may also be any of primary cells, passage cells, and frozen cells. The type of organism used as the source of the cells is not limited, and for example may be the same species as the species to which the therapeutic or prophylactic agent of the present disclosure is administered. Examples include, but are not limited to, mammals for example chimpanzees such as humans and primates; experimental animals such as rats and mice; livestock animals such as pigs, cows, horses, sheep, and goats; and pets such as dogs and cats, for example may be humans or mice, preferably humans.

In the present specification, "treating" includes the complete or partial cure, alleviation, or prevention of progression of the disease in a patient who has already had symptoms or the accompanying symptoms. In the present specification, "preventing" includes the suppression of the onset or the reduction of the morbidity in a patient who is likely to develop the disease.

Those skilled in the art can appropriately determine the amount (for example, a therapeutic and/or preventive effective amount) of exosomes contained in the therapeutic or prophylactic agent of the present disclosure by considering various important factors such as the gender, weight and age of the subject, and progression of disease and symptoms. For example, the amount of exosomes contained in the composition of the present disclosure for example may be, but is not limited to, about 0.0001 to 100.0 mg, or about 0.001 to 10 mg, about 0.01 to 1.0 mg or about 0.05 to 2.0 mg per kg of body weight of the subject to which the composition is administered.

In addition to the above-mentioned exosomes or therapeutic and/or prophylactic agent, the composition of the present disclosure may also contain other ingredients, pharmaceutically acceptable carriers and other carriers, such as sterile water, physiological saline, buffers, excipients, binders, disintegrants, emulsifiers, surfactants, stabilizers, lubricants, diluents, fluidity promoters, flavoring agents, coloring agents, perfumes, etc. The composition of the present disclosure may be formulated by a conventional method. For example, the formulation can refer to the method described in Remington's Pharmaceutical Sciences (Merck Publishing CO., Easton, Pa.).

The administration mode is not particularly limited, and may be appropriately selected according to needs. In general, it can be administered as an oral dosage form such as tablets, capsules, granules, fine granules, powders, liquids, syrups, suspensions, emulsions, and elixirs, or as a non-oral dosage form such as injections, drips, suppositories, inhalants, transdermal absorbents, transmucosal absorbents, sprays, patches, and ointments.

The administration route of the composition of the present disclosure is not limited. For example, it may be administered by inhalation, spray, injection, drip infusion, oral, transdermal, nasal, topical, vaginal or rectal administration. In addition, the composition of the present disclosure can also be formulated into a composition that is administered by inhalation, spray, injection, drip infusion, oral, transdermal, nasal, topical, vaginal or rectal administration. The route of administration may be appropriately selected according to the applicable disease. For example, if it is a lung disease, inhalation or spray administration may be used.

The therapeutic and/or prophylactic agent or composition of the present disclosure can be administered to a subject which includes, but is not limited to, mammals for example chimpanzees such as humans and primates; experimental animals such as rats and mice; livestock animals such as pigs, cows, horses, sheep, and goats; and pets such as dogs and cats, for example humans or mice, preferably humans.

Those skilled in the art can appropriately determine the amount, interval, and duration of administration of the exosomes, therapeutic and/or prophylactic agent, or composition described in this specification by considering various important factors such as the gender, weight and age of the subject, and progression of disease and symptoms. For example, the exosomes, therapeutic and/or prophylactic agent, or composition may be administered in an amount of about 0.0001-100.0 mg/1 kg of body weight, about 0.001-10 mg/1 kg of body weight, about 0.01-1.0 mg/1 kg of body weight, or about 0.05-2.0 mg/1 kg of body weight. In addition, the number of administrations may be, but is not limited to, 3 times a day, twice a day, once a day, once every two days, once every three days, once a week, once every two weeks, once a month, etc. In addition, the administration duration may be, but is not limited to, one day, two days, three days, one week, two weeks, one month, six months, one year or more.

In one embodiment, the present disclosure relates to a method for treating or preventing at least one disease selected from fibrotic diseases, inflammatory diseases, and aging diseases, which comprises administering to a subject the exosomes, the therapeutic or the prophylactic agent described in this specification. In another embodiment, the present disclosure relates to the exosomes, the therapeutic or the prophylactic agent described in this specification for use in the treatment or prevention of at least one disease selected from fibrotic diseases, inflammatory diseases, and aging diseases.

### Example

Hereinafter, the present disclosure will be described in detail using examples, but the scope of the present disclosure is not limited thereto. It should be noted that the documents cited throughout the specification of the present disclosure are used in the specification of the present disclosure by reference in their entireties.

### (Statistical analysis)

The following experiments were repeated at least 3 times, and the results are shown as the mean±SEM. Analysis was performed using Student's t test for comparison among 3 groups or using ANOVA for comparison among more than 3 groups. *p* Value <0.05 was set as a statistically significant difference using Prism version 7 (GraphPad Software, San Diego, CA).

### (Example 1) Collection of exosomes from mesenchymal stem cells

Human MSCs were cultured in STEMPRO MSC SFM medium (THERMOFISHER). Cells were re-inoculated into a subconfluent state when the required number of cells was reached, and cultured in the same medium for 48 hours. Supernatant was collected from a total of 200 ML of culture medium. The collected culture supernatant was filtered through a 0.22 µm filter (MILIPORE). Large particles and fine fragments were observed. Then, exosomes secreted from MSCs were collected and purified by centrifugation (100000×G for 1 hour, BECKMAN). The obtained exosomes were measured for particle system and particle number by using NANOSIGHT (NANOSIGHT) as a nanoparticle tracking system.

### (Example 2) Myofibroblast differentiation inhibition assay in vitro

TGFβ was added to cultured human fibroblasts to experimentally induce differentiation of the cells to myofibroblasts which were used as an *in vitro* model of COPD and IPF produced in the lungs of patients. Human primary lung fibroblasts were obtained by isolating and culturing lung resection tissues at affiliated hospital of Jikei University School of Medicine, Tokyo. The method of isolating and culturing was as reported (Araya J. et aL., 2007, J. CLin. Invest., 117, pp. 3551-3562). The cells were maintained in DMEM medium containing 10% heat-inactivated fetal bovine serum (FBS) and Antibiotic-AntimyCOtic (Thermo Fisher Scientific) at 37 °C, 5% CO₂.

After 24 hours from the start of the culture of fibroblasts, (A) TGFβ (2 ng/mL), (B) TGFβ (2 ng/mL) + MSC-derived exosomes (10 µg/mL), and (C) TGFβ (2 ng/mL) + pirfenidone (PFD) (10 µg/mL) were added, and the expression level (mRNA) of αSMA was quantified after 48 hours.

As a result, addition of MSC-derived exosomes (10 µg/mL) resulted in decreased expression level (mRNA) of αSMA and extremely reduced degree of fibrosis (Fig. 1). Its effect was better than that of pirfenidone, which is a general drug for IPF.

### (Example 3) Evaluation of therapeutic effects using an animal model

Animal experiments were carried out in accordance with the guidelines of the Laboratory Animal Research Institute of the National Cancer Research Center of Japan. 2 U/kg of bleomycin (Nippon Kayaku CO., 4234400D4032) was dissolved in 50 µL of saline and administered via airway to 8-12 week old C57BL/6J mice to prepare mouse models of pulmonary fibrosis for use. 10 µg of exosomes secreted from MSCs were administered through the airway twice, one at 1 week (Day 7) and the other one at another 1 week (Day 14) after bleomycin administration (Day 0). Six days after the second administration of exosomes (Day 20), the mice were euthanized, and the lung was measured for fibrosis score by triple staining. A group not administered with exosomes and a group not administered with neither bleomycin nor exosomes were used as control. 10 mice were used in each group. The administration schedule is shown in Fig. 2.

As reported, the mouse lungs were evaluated by triple staining, haematine and eosin staining (Kobayashi K. et al., J. ImmunoL., (2016) 197(2), pp:504-16). In addition, the triple-stained tissues were evaluated for fibrosis using the Ashcroft's method to determine the Ashcroft score.

The results are shown in Figure 3. The administration of MSC-derived exosomes excellently suppressed lung fibrosis.

## Claims

1. A therapeutic or prophylactic agent for at least one of fibrotic diseases, inflammatory diseases, and aging diseases, comprising exosomes derived from mesenchymal stem cells.

2. The therapeutic or prophylactic agent according to claim 1, wherein
the mesenchymal stem cells are cells isolated from bone marrow, adipose tissue, umbilical cord or peripheral blood.

3. The therapeutic or prophylactic agent according to claim 1, wherein
the mesenchymal stem cells are cells isolated from pluripotent stem cells including iPS cells and ES cells.

4. The therapeutic or prophylactic agent according to any one of claims 1 to 3, wherein
the mesenchymal stem cells are human cells.

5. The therapeutic or prophylactic agent according to any one of claims 1 to 4, wherein
the diseased site of the disease is lung.

6. The therapeutic or prophylactic agent according to claim 5, wherein
the disease is selected from the group consisting of pulmonary fibrosis, COPD, bronchial asthma, ARDS (acute respiratory distress syndrome), pulmonary hypertension, radiation pneumonitis, pulmonary sarcoidosis, alveolar hemorrhage accompanied by collagen disease, or interstitial pneumonia.

7. The therapeutic or prophylactic agent according to any one of claims 1 to 6, which is an inhalant.

8. The therapeutic or prophylactic agent according to any one of claims 1 to 7, which is used for intratracheal administration or transpulmonary administration.
